# EUROPEAN PATENT SPECIFICATION

(11) **EP 3 437 691 B1**
(45) Date of publication and mention of the grant of the patent: **07.07.2021**
(21) Application number: 17774878.7
(22) Date of filing: 27.03.2017
(51) Int. Cl.: A61B 17/34, A61B 17/60

(54) **TOOL FOR SUSTAINED RELEASE OF ANTIBIOTIC**
WERKZEUG ZUR FORTGESETZTEN FREISETZUNG VON ANTIBIOTIKA
OUTIL POUR LIBÉRATION PROLONGÉE D'ANTIBIOTIQUE

(30) Priority: 28.03.2016 JP 2016063474
(43) Date of publication of application: 06.02.2019
(73) Proprietor: Maruo, Akihiro, Himeji-shi, Hyogo 672-8081 (JP)
(72) Inventor: Maruo, Akihiro, Himeji-shi, Hyogo 672-8081 (JP)
(74) Representative: Horn Kleimann Waitzhofer Patentanwälte PartG mbB
(86) International application number: PCT/JP2017/012279
(87) International publication number: WO 2017/170341

(56) References cited:
- WO-A1-2008/079550
- JP-A- 2001 212 151
- JP-A- 2010 510 002
- JP-A- 2015 509 396
- JP-A- 2015 517 879
- US-A- 5 681 289
- US-A1- 2013 261 650
- SHINSUKE KIHARA et al.: "Hone Kansensho ni Taisuru Kotsuzui Tenteki no Yakubutsu Dotai", Journal of Japanese Society for Fracture Repair, vol. 37, no. 1, 5 February 2015 (2015-02-05), pages 228-230, XP009511487, ISSN: 0287-2285

## Description

### Technical Field

This invention relates to a device for intra medullary antibiotics perfusion (iMAP) which can perfuse antibiotics continuously to a patient's bone fracture surgical site for suppressing the occurrence of post traumatic deep surgical site infection (SSI). For example, iMAP can perfuse prescribed dose antibiotics continuously from outside to the patient's bone fracture surgical site like an intravenous drip.

### Background Art

In an orthopedic field, It is very important to take care and suppress the occurrence of the post traumatic deep surgical site infection (SSI) and various complications. It is very important to keep on curing even after finishing the bone fracture surgical operation especially in the case of the traumatic bone fracture with open wound fracture because there is a risk of the bacteria invasion into the tissue.

An effective treatment for suppressing the occurrence of post traumatic deep surgical site infection (SSI) is a local drug delivery care of antibiotics to the patient's surgical site. However, the bone tissue and the peripheral tissue around the bone fracture surgical site is not easy to suppress the occurrence of post traumatic deep surgical site infection (SSI). The reasons are described below.
1. The blood flow becomes poor because the bone tissue and the peripheral tissue are damaged by the huge force in the accident.
2. The blood and body fluid don't flow well and easy to stagnate at the damaged portion.
3. Antibiotics is hardly distributed to the patient's vascularized tissue by an intravenous drip method because the fine vascularized tissue is damaged too.
4. A bio-film (aggregation of bacteria) is formed around the metal tool for fixing the bone fracture surgical site. It becomes barrier against antibiotics flow.

The method for delivering antibiotics should be selected by taking into consideration the above-mentioned circumstances. In the prior art, there are 3 methods as the conventional antibiotics delivery methods.
1. Oral administration method = A preparation for internal use
2. Drip infusion method = Intravenous injection
3. Local delivery method = Indwell in the target portion by mixing antibiotics and bone cement

Even though the drip infusion method via the vascularized tissue can deliver the antibiotics to the target portion better comparing with the oral administration method, but the antibiotics will deliver to whole body around by either the oral administration method or the drip infusion method. Therefore, there is a limit on the dose level of the antibiotics. If the high dose antibiotics is delivered to the bone fracture treatment site by either the oral administration method or the drip infusion method, other organ may suffer adverse influence from being exerted to the high dose antibiotics delivered by the blood around whole body. In contrast, the delivery range of antibiotics by the local delivery method is limited around the patient's therapeutic tissue, so the local delivery method has advantage on the dose level of the antibiotics with little influence on other organs.

In the prior art, there are several conventional inventions for perfusing antibiotics continuously to a patient's bone fracture surgical site are known as follows.

For example, JP 2015-509396 is known as shown by Fig.18. This invention utilizes implant tool for being embedded into the patient's therapeutic bone tissue in the bone fracture surgical site. It describes a system for perfusing the requested agent such as antibiotics by enclusing in the implant which provides a scaffold for bone regeneration and the base for perfusing the antibiotics. The portion where the implant is embedded is the bone fracture surgical site, so this invention can perfuse the antibiotics around the patient's therapeutic bone tissue.

For example, JP 2015-517879 is known as shown by Fig.19. This invention utilizes 3-dimention implant. 3-dimention implant is manufactured by the multi-layer 3-dimention printer with the applicable material for embedding into the bone tissue. 3-dimention implant is made by permeable porous structure and has hollow space where the medical agent such as antibiotics and growth agent are contained. If the 3-dimension implant is applied to the bone fracture surgical site, it can hold the antibiotics and perfuse the antibiotics around the bone fracture surgical site.

Prior art 1: JP 2015-509396
Prior art 2: JP 2015-517879
Attention is drawn to WO 2008/079550 A1 which relates to an external fixation assembly which includes a plurality of hollow pins that are inserted into a patient's bone. Each pin has an interior bore and a plurality of apertures extending through the pin wall from the bore. The pin is coupled to a source of vacuum pressure operable to create reduced pressure in the tissue surrounding the pin. A cover is placed around the pin and sealed to provide a fluid-tight enclosure that maintains reduced pressure around the pin. The method for applying external fixation using the fixator pins includes the steps of inserting each pin through a skin opening, positioning the pin apertures near selected tissue, covering the skin opening with a sealed enclosure, connecting the pins to a source of vacuum pressure, and activating the source of vacuum pressure.

### Disclosure of the invention

### The problems to be solved

Whether the conventional device for perfusing antibiotics in the prior art faces problems as follows.

The first problem is that the conventional device is embedded type, so the indwell state in the target portion maintains for a long term during the curing period. The dose level and the interval of antibiotics administration should be adjusted and changed according to the circumstances of the state of tissue. However, the conventional device is embedded into the target portion, so post operation is requested every time if the antibiotics administration should be changed in its kind and its dose level in the embedded device. Moreover, the administration interval cannot be changed because the conventional device always perfuses antibiotics constantly.

The second problem is that the dose level adjustment is not possible even though the administration dose level perfused by the conventional device is gradually weakened. If the administration dose level is an appropriate dose level at the beginning of the embedding operation of the implant, the perfuse dose level becomes gradually weaken. If the initial administration dose level of the antibiotics is adjusted higher by considering the weakened pace of the administration dose level, the initial administration of the antibiotics causes overdose, living body load of the patient becomes large.

As shown above problems, a new device is requested for providing a scaffold for perfusing antibiotics besides the bone fracture surgical site without indwelling in the target portion, which new device employs structure accessible easily from the outside and can perfuse appropriate antibiotics according to the circumstances of tissue in the bone fracture surgical site.

It is an object of the present invention to provide such a new device for providing a scaffold for perfusing antibiotics besides the bone fracture surgical site, which new device employs structure accessible easily from the outside and can perfuse appropriate antibiotics according to the status of the bone fracture surgical site according to the circumstances of tissue in the bone fracture surgical site. These characteristics are difficult to given by the conventional device in the prior art.

Means for solving the problems

In order to achieve the above-mentioned object, the present invention provides a device for intra medullary antibiotics perfusion (iMAP) for perfusing an antibiotics to a patient's bone fracture surgical site, applied as an external skeletal fixator as set forth in claim 1.

According to the above-mentioned configuration, the present invention iMAP can provide a scaffold for perfusing antibiotics besides the bone fracture surgical site and can perfuse appropriate kind and dose level of antibiotics according to the circumstances of tissue in the bone fracture therapeutic portion.

The present invention iMAP can provide appropriate kind and dose level of antibiotics via a transfusion tube according to the circumstances of tissue in the bone fracture therapeutic portion.

At least, the iMAP has two phases for connecting to the external tools. The first phase is an electric drill connection phase for rotating and fixing the iMAP to the bone besides the bone fracture surgical site. In this first phase, the iMAP should be connected firmly to the drill chuck of the electric drill to rotate. The second phase is a transfusion tube connecting phase for perfusing antibiotics. In this second phase, the iMAP should be connected to the lock connector attached on the head of the transfusion tube.

As shown above, the present invention iMAP may connect to the external tools in these two phases. Therefore, there are two patterns for connecting procedures.

The first pattern is that a transfusion tube connector which shape can fit to the lock connector on the head portion of the external antibiotics transfusion tube is installed to the base end part of the iMAP, the iMAP can connect to the antibiotics transfusion tube via the transfusion tube connector.

According to the first pattern, the transfusion tube connector is provided as a constant element attached to the iMAP, so the antibiotics transfusion tube can connect easily in the transfusion tube connecting phase.

In this first pattern, a drill attachment is included for providing a structure connecting to a drill chuck of an electric drill tool, which is used in the electric drill connecting phase.

The drill attachment comprises a transfusion tube adapter for connecting to the transfusion tube connector on the base end part of the fixation pin, wherein the adapter shape is the same as that of the lock connector on the head part of the transfusion tube, and a chuck connecter which can be chucked by the drill chuck of the electric drill tool.

By utilizing the drill attachment, the shape of the base end part of the fixation pin can be converted to the shape applicable to the electric drill tool by the drill attachment.

The second pattern is that a drill attachment is included for providing a structure connecting to a drill chuck of an electric drill tool installed to the base end part of the iMAP, so the iMAP can connect to the external electric drill tool via the chuck of the electric drill tool.

According to the second pattern, the drill attachment is provided as a constant element attached to the iMAP, so the external electric drill tool can connect easily in the electric drill connecting phase.

In this second pattern, a transfusion tube attachment is included for providing a structure connecting to the lock connector of the external transfusion tube in the transfusion tube connecting phase.

The transfusion tube attachment comprises a drill connector adapter for connecting to the drill connector and a transfusion tube connector for connecting to the lock connector on the head part of the transfusion tube.

By utilizing the transfusion tube attachment, the shape of the base end part of the fixation pin can be converted to the shape applicable to the lock connector of the external transfusion tube.

It is preferable that the transfusion tube attachment includes a detachable mechanism which can adjust the gripping force between the inner surface of the drill connector adapter and the outer surface of the drill connector of the fixation pin.

For example, the detachable mechanism comprises a gripping lock ball installed facing to the outer surface of the drill connector of the fixation pin; an elastic actuator; a gripping force adjuster for adjusting the gripping force corresponding to the pressure generated between the gripping lock ball and the outer surface of the drill connector when sliding along a track by the elastic actuator; wherein the structure of the gripping force adjuster contacting to the gripping lock ball has a wedge-shape. By this configuration, the gripping force adjuster moves along to the track by the elastic actuator, the gripping force to the drill connector of the fixation pin by the gripping lock ball becomes large by the work of the wedge-shape to the gripping lock ball.

Next, it is preferable that the outer shape of the fixation pin is the same as the outer shape of a conventional pin for an external skeletal fixation applied to patient's bone fracture surgical site. By this configuration, the iMAP can apply as the external skeletal fixator providing both external skeletal fixation function and intra medullary antibiotics perfusion function to patient's bone fracture surgical site.

Next, it is preferable that the fixation pin has a wider portion which diameter is larger than that of other portion. If the patient's bone fracture is a complicated bone fracture, plural fixation pins are fixed to plural points besides patient's bone fracture surgical site, plural fixation pins are combined as a cluster. The present invention iMAP can provide a bridge connector for connecting between plural fixation pins even if there is a variety in diameter. The bridge connector can connect easily because the fixation pin of the iMAP has a wider portion which diameter is larger than that of other portion.

Antibiotics agent can be perfused from at least one of the device for intra medullary antibiotics perfusion among the cluster.

### Effect of the invention

According to the device for intra medullary antibiotics perfusion (iMAP) of the present invention, the present invention iMAP can provide a scaffold for perfusing antibiotics besides the bone fracture therapeutic portion and can perfuse appropriate kind and dose level of antibiotics according to the circumstances of tissue in the bone fracture therapeutic portion.

### Brief description of the drawings

Figs. 1-6 are schematic views of a first embodiment of a device for intramedullary antibiotics perfusion.
Figs. 7-14 are schematic views of a second embodiment of a device for intramedullary antibiotics perfusion.
Figs. 15-17 are schematic views of third embodiments of a device for intramedullary antibiotics perfusion.

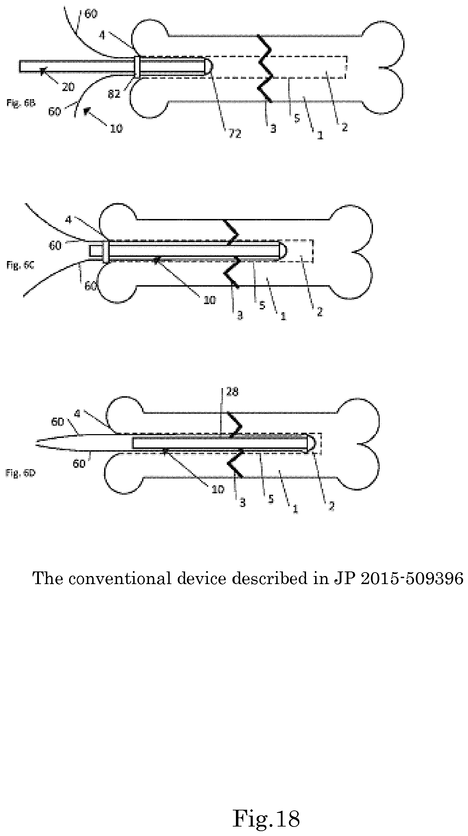

Fig. 18 is a schematic view of a conventional device described in JP 2015-509396.

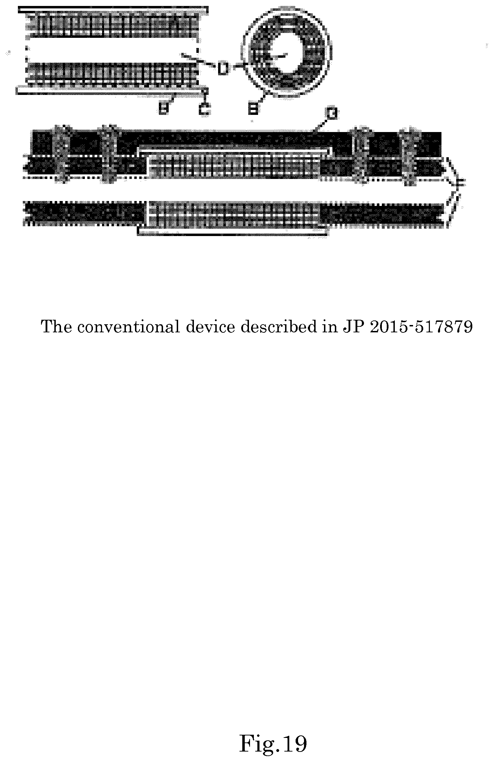

Fig. 19 is a schematic view of a conventional device described in JP 2015-517879.

### Detailed description of the preferred embodiment

Some embodiments of a device for intra medullary antibiotics perfusion according to the present invention are described below with reference to the relevant drawing. Needless to add, the claims of the present invention include but are not limited to the application, configuration, or quantity shown in the following embodiments.

Hereinafter, the configuration in which the fixation pin (iMAP pin) 110 and the transfusion tube connector 120 are combined as one body is described in Embodiment 1, the configuration in which the fixation pin (iMAP pin) 110 and the drill attachment 150 are combined as one body is described in Embodiment 2, the example of the configuration applied to the an external skeletal fixation of the patient's bone fracture surgical site is described in Embodiment 3.

### Embodiment 1

The device for intra medullary antibiotics perfusion (iMAP) 100 of embodiment 1 according to the present invention is described with referencing to the drawings.

The procedure for applying iMAP has two phases for connecting to the external tools. The first phase is an electric drill connecting phase and the second phase is a transfusion tube connecting phase. In this Embodiment 1, the transfusion tube connector 120 used in the transfusion tube connecting phase is provided as a constant element, and the drill connector used in the electric drill connecting phase is provided as an attachment.

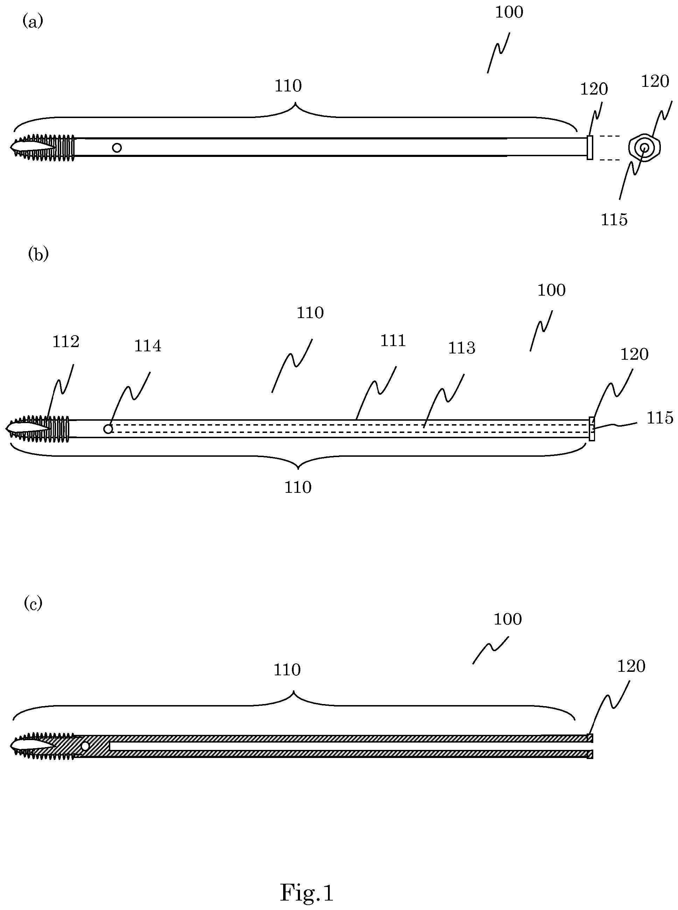

Fig.1 is a schematic view of the structure of the device for intra medullary antibiotics perfusion (iMAP) 100.

As shown in Fig.1 (a), the iMAP 100 includes a fixation pin (iMAP pin) 110, and a transfusion tube connector 120. Fig.1 (b) is a figure for describing the elements of the iMAP 100. In Fig.1 (b), a hollow portion 113 as an inner structure is shown by broken line to recognize the inner structure easily.

In this Embodiment 1, the fixation pin (iMAP pin) 110 and the transfusion tube connector 120 are formed as one body and cannot be separated each other.

Each element of the fixation pin (iMAP pin) 110 is described below.

The fixation pin (iMAP pin) 110 is an element for externally fixing to a bone besides the bone fracture surgical site. The fixation pin (iMAP pin) 110 comprises a shaft 111, a screw part 112, a hollow portion 113, a side face opening 114 and a base end opening 115.

The shaft 111 is a rod shape pin made from stainless steel or titanium metal having a screw pin outer shape. The surface treatment with a hydroxyapatite coating is preferable for stable fixation.

The shaft diameter, whole length, and partial length of the screw part are variety according to the shape and the largeness of the bone where the fixation pin (iMAP pin) 110 is applied. For example, whole length is 150 mm, outer diameter is 5.0 mm, and partial length of the screw part is 15 mm. The base end shape is a stryker-Hoffmann type. It is provided as a disposable element.

The screw part 112 is a male screw portion including spiral screw head mounted on the outer circumferential surface of the tip part of the fixation pin. The screw part 112 is provided as a portion to be screwed into the bone. The screw portion is a variety in its pitch according to the shape and the largeness of the bone where the fixation pin (iMAP pin) 110 is applied. For example, the male screw of this configuration has notched portion for assisting the fixation pin to be able to screw into the harder bone tissue with small damage.

As the material and the shape for the shaft 111 and the screw part 112, the same material and the shape for the general fixation pin used as the external fixator for bone fracture can be employed. The general fixation pin used as the external fixator for bone fracture is widely spread and generally used in orthopedics field for bone fracture treatment. The general fixation pin is a medical device which operative method for fixing to the bone is established as the general external skeletal fixator. A lot of information about the general fixation pin is accumulated. It is convenient for the shaft 111 and the screw part 112 of the present invention iMAP to employ the same material and the shape for the general external fixator because the shaft 111 and the screw part 112 of the present invention iMAP is the same as the general fixation pin in view of the purpose that the fixing element for piercing into the bone besides the bone fracture surgical sit.

Next, the hollow portion 113 is an inner space from the base end opening 11 to the side face opening 114. As described later, antibiotics is perfused through the hollow portion 113 to the patient's bone fracture surgical site. The inner shape of the hollow is not limited to particular shape. It is a simple cylindrical shape in this example.

The side face opening 114 is an opening on the side surface of the shaft 111 of the fixation pin (iMAP pin) 110 and is connected through the hollow portion 113. The side face opening 114 is located facing to the bone marrow of the bone besides the bone fracture portion after fixing treatment. As described later, antibiotics is perfused to the patient's bone fracture surgical site only via the side face opening 114.

The base end opening 115 is located at the base end part of the fixation pin (iMAP pin) 110 and is connected through the hollow portion 113. As described later, the base end opening 115 works as an acceptable opening for antibiotics transfused from the external transfusion tube via the transfusion tube connector 120. In this configuration, the fixation pin (iMAP pin) 110 and the transfusion tube connector 120 are provided as one body, the base end opening 115 is connected to the base end opening of the transfusion tube connector 120 directly.

It is preferable that the screw part 112, the hollow portion 113, the side face opening 114 and the base end opening 115 are made from stainless steel or titanium metal, and the surface treatment with a hydroxyapatite coating are provided for stable fixation.

The whole shape of the fixation pin (iMAP pin) 110 is the same that of the general fixation pin used for the external skeletal fixator except for the original structure of the side face opening 114 on the side surface near the head portion and the base end opening 115 on the bottom end portion and hollow portion 113 through from the side face opening 114 to the base end opening 115.

Next, the transfusion tube connector 120 used in the phase for perfusing antibiotics is described below.

In the configuration of the iMAP 100 in Embodiment 1, the transfusion tube connector 120 is provided on the base end portion of the fixation pin (iMAP pin) 110, so antibiotics is transfused from the external transfusion tube 200 via the transfusion tube connector 120.

The method for connecting the transfusion tube connector 120 and the external transfusion tube 200 is not limited. For example, an adapter or socket may be employed. If the outer diameter of the one element can fit to the inner diameter of the other element, a plug-in connector can be employed and fixing them by wrapped around with sealing tape. It is preferable to treat these elements carefully so as not to invade bacteria from this connecting portion because antibiotics is transfused through these elements.

In this example, the shape of the transfusion tube connector 120 is the shape which can fit to the lock connector 210 on the head portion of the external transfusion tube 200.

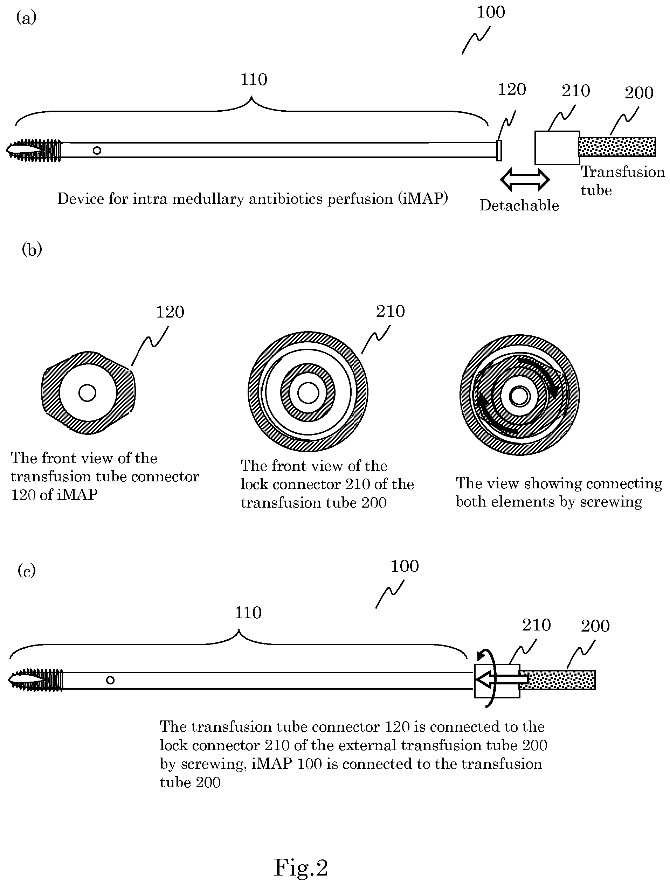

Fig.2 is a schematic view showing the lock connector 210 of the external transfusion tube 200 is connected to the transfusion tube connector 120 of the iMAP 100 of Embodiment 1.

As shown in Fig.2, the transfusion tube connector 120 of the iMAP 100 is connected to the lock connector 210 of the external transfusion tube 200 filled with antibiotics. Antibiotics is transfused from the external transfusion tube 200 to the base end opening 115 of the fixation pin (iMAP pin) 110 and the antibiotics is perfused from the side face opening 114 through the hollow portion 113.

As shown in Fig.2, the transfusion tube connector 120 includes the male screw which fit to the inner female screw of the lock connector 210. The transfusion tube connector 120 can be connected to the lock connector 210 by screwing; the fixation pin (iMAP pin) 110 and the external transfusion tube 200 can be connected firmly.

Next, the procedure for connecting the drill attachment 140 in the electric drill connecting phase is described below.

The drill attachment 140 is an attachment for providing the element which can connect to a drill chuck 310 of an electric drill tool 300.

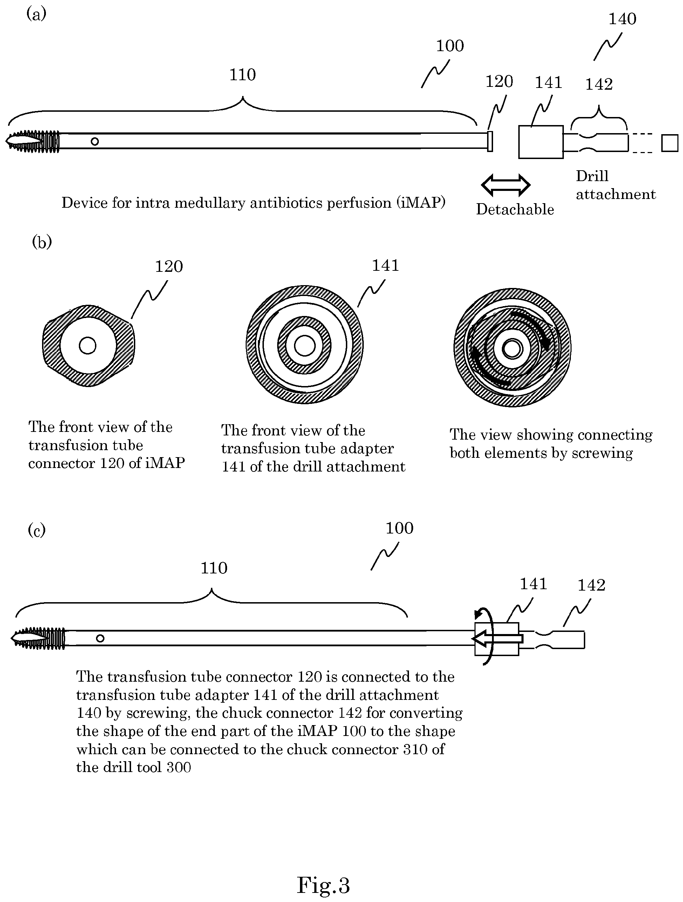

Fig.3 is a schematic view of the structure of the drill attachment 140 and view showing the drill attachment 140 is connected to the transfusion tube adapter 141 of the iMAP 100 of Embodiment 1.

As shown in Fig.3, the drill attachment 140 comprises a transfusion tube adapter 141 and a chuck connecter 142.

The transfusion tube adapter 141 has the same shape as that of the lock connector 210, so it can connect to the transfusion tube connector 120 on the base end part of the fixation pin (iMAP pin) 110.

As shown in Fig.3 (b) and Fig.3 (c), the transfusion tube connector 120 includes the male screw which fit to the inner female screw of the transfusion tube adapter 141. The transfusion tube connector 120 can be connected to the transfusion tube adapter 141 by screwing. As a result, the fixation pin (iMAP pin) 110 and the drill attachment 140 can be connected firmly.

When the drill attachment 140 is connected to the fixation pin (iMAP pin) 110, a chuck connecter 142 is provided at the end of the fixation pin (iMAP pin) 110. Therefore, the shape of the base end of the iMAP can converted to the shape which can connect to the drill chuck 310 of an electric drill tool 300.

The chuck connecter 142 is an element having the shape which can be chucked by the drill chuck 310 of the electric drill tool 300. The shape of the chuck connecter 142 is not limit to the particular shape if it can fit to the drill chuck 310 of the electric drill tool 300. In this example, the chuck connecter 142 includes an element which cross sectional shape is a predetermined size square shape fit to the drill chuck 310; and the dimple portion in the inner wall, which dimple can accept a ball installed to the drill chuck 310 of an electric drill tool 300.

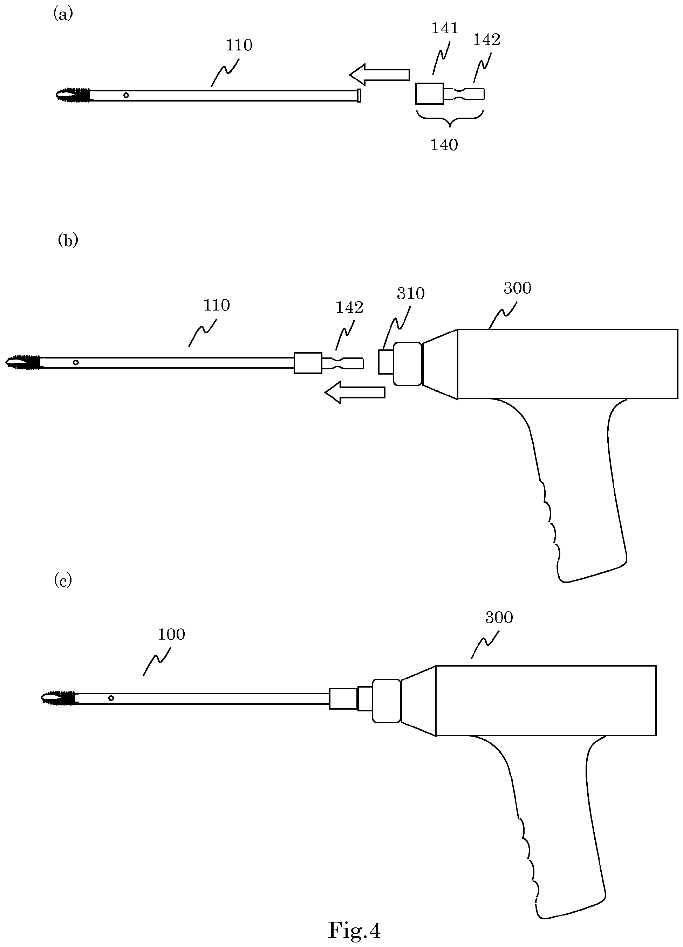

Next, the operation procedure of the iMAP 100 of Embodiment 1 is descried below with referencing to Fig.4 to Fig.6.

As shown in Fig.4 (a), the drill attachment 140 is attached to the base end portion of the fixation pin (iMAP pin) 110 for preparing the electric drill connection phase as shown in Fig.3.

As shown in Fig.4 (b) to Fig.4 (c), the drill chuck 310 of an electric drill tool 300 is connected to the chuck connecter 142 attached to the base end portion of the iMAP 100. The external electric drill tool 300 is connected to the iMAP 100.

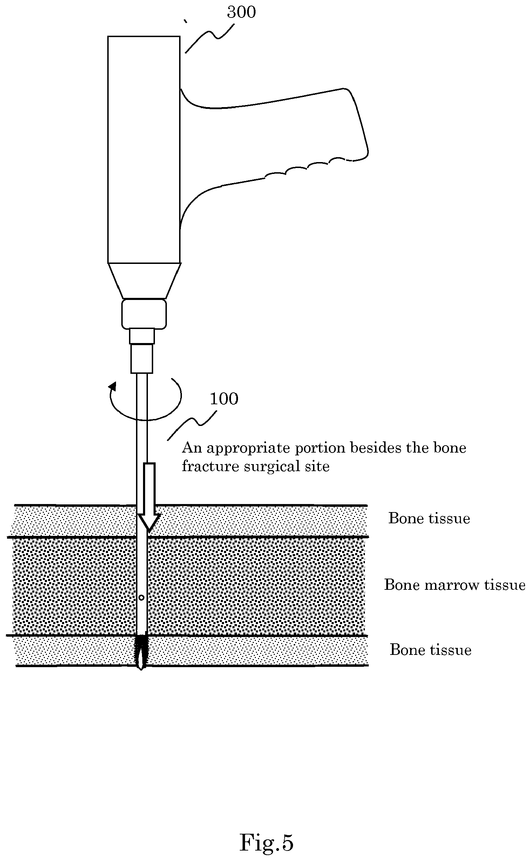

Next, As shown in Fig.5, the iMAP 100 connected to the electric drill 300 of Embodiment 1 is fixed by piercing to the bone besides the patient's bone fracture surgical site. In this Embodiment 1, an operation for a single iMAP 100 is described. An operation for a plurality set of iMAP 100 is described in Embodiment 3.

The fixation pin (iMAP pin) 110 has male screw 112 on the head portion for screwing. In advance to pierce the fixation pin (iMAP pin) 110 to the target bone portion, it is preferable that an assist hole which assists the beginning of screwing is opened beforehand by the electric drill tool. This assist hole can assist the fixation pin to be screwed and pierced even if the target bone portion is hard bone tissue. The size of the assist hole should be adjusted to fit the fixation pin.

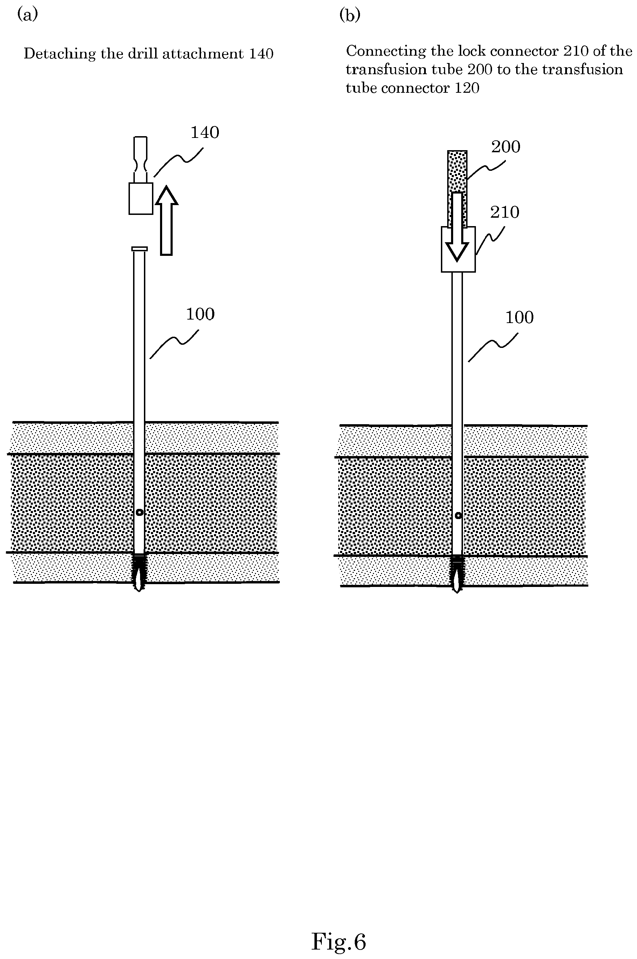

After fixing the fixation pin (iMAP pin) 110 to the appropriate portion according to the same operation as that of the external skeletal fixator, the electric drill tool and the drill attachment 140 are detached as shown in Fig.6 (a). As a result, the iMAP 100 is left in the patient's bone fracture surgical site. In this state, the side face opening 114 is adjusted to face to the marrow of the patient's bone fracture surgical site.

A transfusion bag containing appropriate kind and dose level of antibiotics is prepared on the appropriate timing during operation; the transfusion tube 200 is connected to the transfusion tube connector 120 via the lock connector 210 as shown in Fig.6 (b).
Appropriate kind and dose level of antibiotics is perfused to the patient's bone fracture surgical site by the predetermined term and the predetermined speed via the route from the transfusion tube 200 - the lock connector 210 - base end opening 115 - the hollow portion 113 - the side face opening 114.

The basic structure and function of the iMAP 100 and the each element are described as shown above. The iMAP 100 of the present invention is applied to the patient's bone fracture surgical site, it is preferable that a required sterilization should be treated on the iMAP as a medical use device.

### Embodiment 2

The device for intra medullary antibiotics perfusion (iMAP) 100a of embodiment 2 according to the present invention is described with referencing to the drawings.

The iMAP has two phases for connecting to the external tools. The first phase is an electric drill connecting phase and the second phase is a transfusion tube connecting phase. The configuration of Embodiment 2, the drill connector 150 used in the electric drill connecting phase is provided as a constant element, and the transfusion tube attachment 160 used in the transfusion tube connecting phase is provided as an attachment.

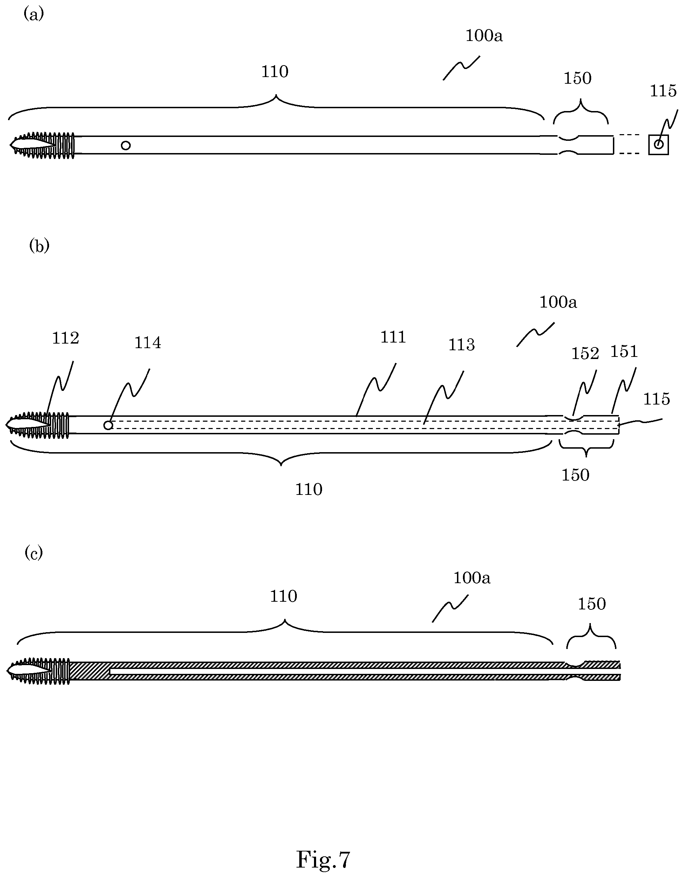

Fig.7 is a schematic view of the structure of the device for intra medullary antibiotics perfusion (iMAP) 100a of this Embodiment 2.

As shown in Fig.7 (a), the iMAP 100a includes a fixation pin (iMAP pin) 110, and a drill connector 150. Fig.7 (b) is a figure for describing the elements of the iMAP 100a. In Fig.7 (b), a hollow portion 113 as an inner structure is shown by broken line to recognize the inner structure easily.

In this Embodiment 2, the fixation pin (iMAP pin) 110 and the drill connector 150 are formed as one body and cannot be separated each other.

As shown in Fig.7, the fixation pin (iMAP pin) 110 comprises a shaft 111, a screw part 112, a hollow portion 113, a side face opening 114 and a base end opening 115.

Regarding the shaft 111, the screw part 112, the hollow portion 113, and the side face opening 114 are the same as shown in Embodiment 1, so the description for those are omitted here.

Antibiotics is delivered through the hollow portion 113. Therefore, the hollow portion 113 should penetrate to the base end opening 115 for introducing the transfused antibiotics. As shown in Fig.7 (c), in this Embodiment 2, the drill connector 150 is formed on the base end portion of the fixation pin (iMAP pin) 110. Therefore the hollow portion 113 penetrates through the drill connector 150, and the base end opening 115 is installed in the end portion of the drill connector 150. From a different point of view, the drill connector 150 has a hollow, and this hollow is connected through the hollow portion 113 in the fixation pin (iMAP pin) 110.

It is preferable that the element of the fixation pin (iMAP pin) 110 is made from stainless steel or titanium metal, and the surface treatment with a hydroxyapatite coating is treated.

The connecting operation of the lock connector 210 of the external transfusion tube 200 and the transfusion tube connector 120 in the antibiotic perfusion phase of the iMAP 100a of Embodiment 2 is described below.

The transfusion tube attachment 160 is an attachment for providing a structure for connecting to the lock connector 210. The transfusion tube attachment 160 can convert the shape of the base end part of the iMAP 110a to the shape applicable to the lock connector 120 on the head part of the transfusion tube 200.

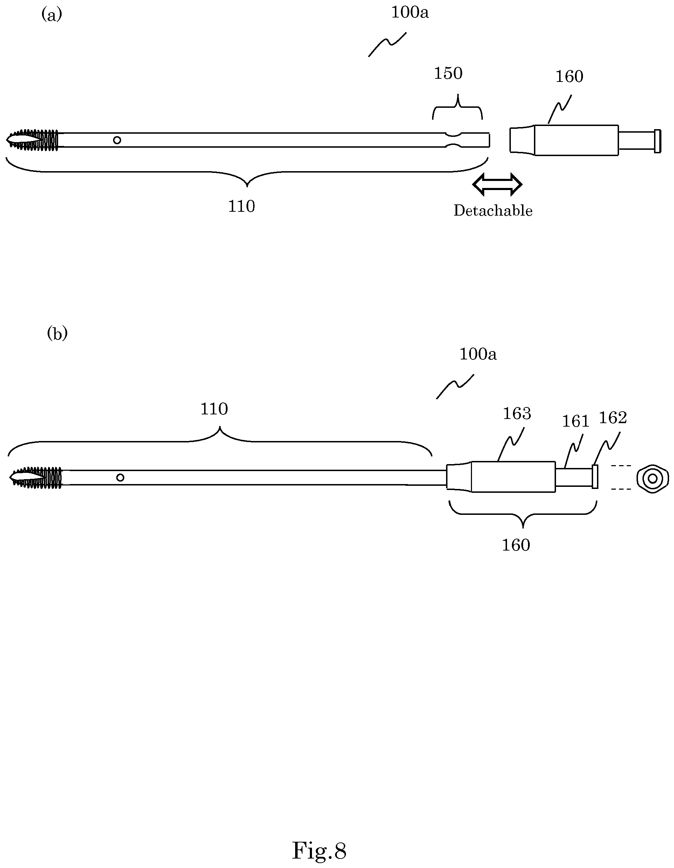

Fig.8 is a schematic view showing the transfusion tube attachment 160 is connected to the drill connector 150 of iMAP 100a of Embodiment 2. As shown in Fig.8, the transfusion tube attachment 160 comprises a drill connector adapter 161 and a transfusion tube connector 162.

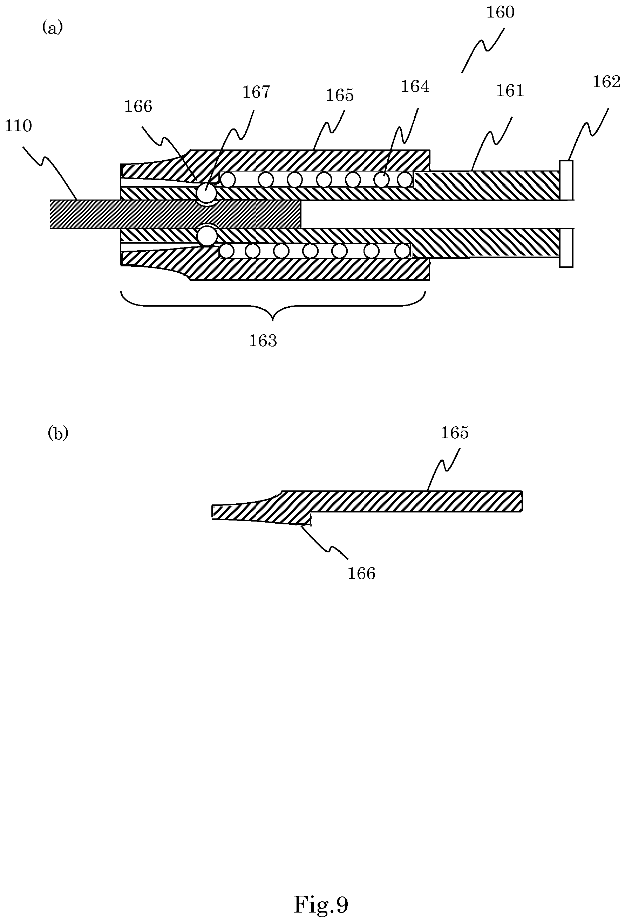

Fig.9 is a schematic view of the structure of the transfusion tube attachment 160. It is an enlarged cross-sectional drawing in the longitudinal section to show the inner structure.

Fig.9 (b) is a schematic view extracting the upper portion of a clipping force adjuster 165 to show a taper structure 166.

As shown in Fig.8 and Fig.9, the transfusion tube attachment 160 comprises the drill connector adapter 161 and the transfusion tube connector 162 and the detachable mechanism 163. In this example, the detachable mechanism 163 comprises an elastic actuator 164; a gripping force adjuster 165; a taper structure 166; and a gripping lock ball 167.

The drill connector adapter 161 is an adapter for connecting to the drill connector 150. The shape of the drill connector adapter 161 is the same as that of the drill chuck310 of an electric drill tool 300. The drill connector 150 originally has the shape fitting to the drill chuck 310 of an electric drill tool 300, so the drill connector 150 can fit to the drill connector adapter 161 which shape is the same as that of the drill chuck 310.

The transfusion tube connector 162 has a shape for connecting the lock connector 210 of the transfusion tube 200. The connection method for connecting the transfusion tube connector 162 and the transfusion tube 200 is not limited. In this Embodiment 2, the transfusion tube connector 162 is the same as that of Embodiment 1.

The detachable mechanism 163 can connect and detach the transfusion tube attachment 160 and the drill connector 150 formed on the fixation pin (iMAP pin) 110. Any mechanism can be employed if it can connect and detach the transfusion tube attachment 160 and the drill connector 150 as the detachable mechanism 163.

In this example, the detachable mechanism 163 adjusts the gripping force corresponding to the pressure generated between the gripping lock ball 167 and the outer surface of the drill connector 150. When the gripping force to the drill connector 150 by the gripping lock ball 167 becomes large, the transfusion tube attachment 160 is pressed to the drill connector 150. When the gripping force to the drill connector 150 by the gripping lock ball 167 becomes small, the transfusion tube attachment 160 is released from the drill connector 150 and detached.

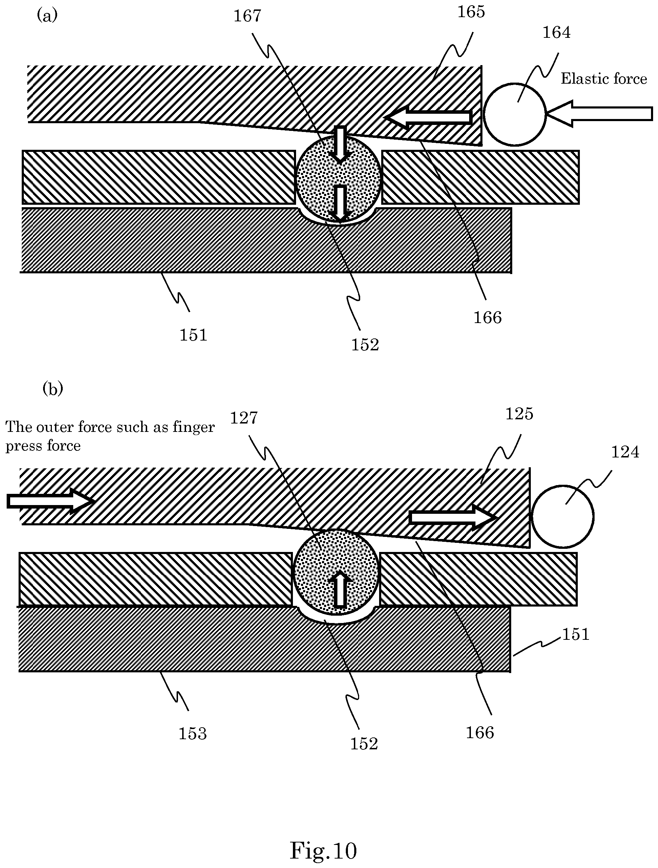

Fig.10 is a schematic view showing the work of the detachable mechanism especially focusing on the work of the gripping force adjuster 165 and the taper structure 166.

As shown in Fig.10, the detachable mechanism 163 includes the gripping lock ball 167.

The gripping lock ball 167 is installed facing to the outer surface of the fixation pin (iMAP pin) 110. As shown in Fig.10, a dimple portion 152 is formed on the inner surface of the drill connector 150 facing to the gripping lock ball 167.

By this configuration, the gripping force becomes larger when the gripping lock ball 167 is pressed stronger to the dimple portion 152. As shown in Fig.8 (b), the transfusion tube attachment 160 is gripped to the drill connector 150 via the gripping lock ball 167 as one body.

The gripping force becomes smaller when the gripping lock ball 167 is pressed weaker to the dimple portion 152. When the gripping force is not enough to grip the transfusion tube attachment 160, the transfusion tube attachment 160 is detached from the fixation pin (iMAP pin) 110.

The detachable mechanism 163 adjusts the gripping force of the gripping lock ball 167.

In this configuration, as shown in Fig.9, the detachable mechanism 163 comprises the elastic actuator 164; the gripping force adjuster 165; the taper structure 166; and the gripping lock ball 167.

Any elastic mechanism such as a spring, a rubber and the mechanism utilizing the magnetic force can be employed as the elastic actuator 164. In this configuration, the inner space between the drill connector adapter 161 and the gripping force adjuster 165 is used for the stable work.

The gripping force adjuster 165 is an element for sliding along by the elastic actuator 164. The gripping force adjuster 165 slides by pressing the gripping lock ball 167.

The sliding method for sliding the gripping force adjuster 165 is not limited. For example, the gripping force adjuster 165 is assembled with the drill connector adapter 161, the sliding orbit of the gripping force adjuster 165 is set along the drill connector adapter 161.

In this example, the taper structure 166 to press the gripping lock ball 167 is employed as a part of the gripping force adjuster 165. The taper structure 166 has a skew that the head direction pushed by the elastic actuator 164 is thinner and the bottom portion attached to the gripping force adjuster 165 is thicker. Therefore, the taper structure 166 presses down the gripping lock ball 167 to the dimple portion 152 formed on the inner surface of the drill connector 150.

Fig.10 is a schematic view showing the work of the gripping force adjuster 165 having the taper structure 166.

Fig.10 (a) is a schematic view showing the work of the gripping force adjuster 165 sliding to the left in the drawing view by the elastic force of the elastic actuator 164 and pressing down the gripping lock ball 167.

As shown in Fig.10 (a), the elastic force of the elastic actuator 164 is applied on the gripping force adjuster 165 to slide to the left. The taper structure 166 of the gripping force adjuster 165 goes to left contacting on the gripping lock ball 167 from the thinner portion. The gripping lock ball 167 is pressed down by the inner surface of the taper structure 166 (which is a part of the inner surface of the gripping force adjuster 165). The more the taper structure 166 goes to left, the larger the pressing force to the gripping lock ball 167 becomes. The gripping lock ball 167 also contacts on the dimple 152 in the electric drill connector 150, the pressing force to the electric drill connector 150 by the gripping lock ball 167 becomes large. As a result, the friction between the electric drill connector 150 and the gripping lock ball 167 becomes large. The transfusion tube attachment 160 is connected to the electric drill connector 150 via the gripping lock ball 167 and is held stably at an appropriate balancing position.

Fig.10 (b) is a schematic view showing the work of the gripping force adjuster 165 sliding to the right in the drawing view by the outer force such as finger press force against the elastic force of the elastic actuator 164 and releasing the gripping lock ball 167.

As shown in Fig.10 (b), the outer force such as the finger press force is applied on the gripping force adjuster 165 to slide to the right. If the outer force overcomes the elastic force of the elastic actuator 164 and is enough for sliding the gripping force adjuster 165 to the right, the gripping force adjuster 165 begins to slide to the right. Then, the taper structure 166 of the gripping force adjuster 165 goes to right. The pressing force to the gripping lock ball 167 by the inner surface of the taper structure 166 (which is a part of the inner surface of the gripping force adjuster 165) becomes small. The more the taper structure 166 goes to the right, the smaller the pressing force to the gripping lock ball 167 becomes. As a result, the grip force between the electric drill connector 150 and the gripping lock ball 167 becomes small, the transfusion tube attachment 160 is detached from the electric drill connector 150.

By this reversible operation as shown by Fig.10 (a) and Fig.10 (b), the transfusion tube attachment 160 can attach to and detach from the electric drill connector 150.

Next, the attachment procedure of the lock connector 210 of the transfusion tube 200 is described. The lock connector 210 is attached after the attaching the transfusion tube attachment 160.

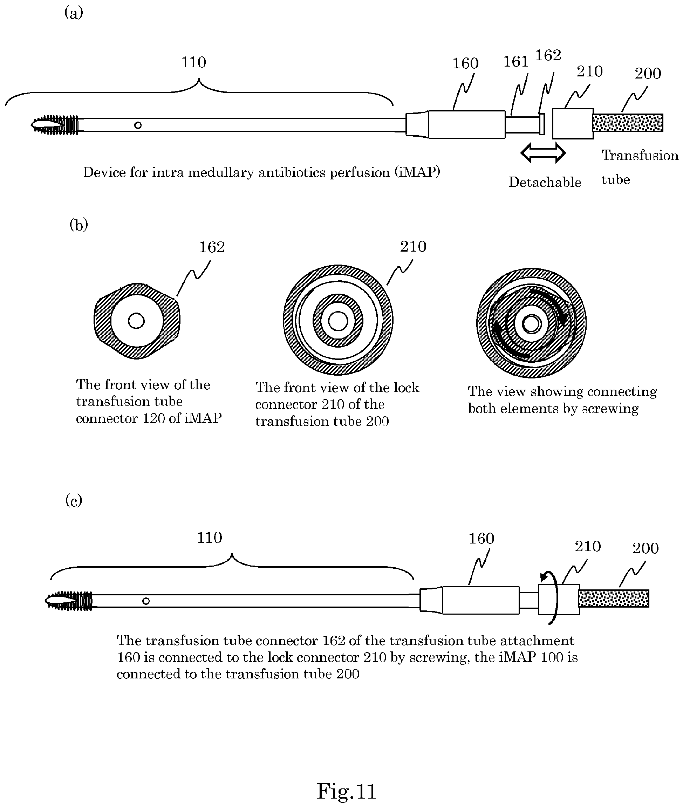

Fig.11 is a schematic view showing the attachment procedure of the lock connector 210 of the transfusion tube 200 connecting to the transfusion tube connector 162 on the base end of the transfusion tube attachment 160.

In this example, the shape of the transfusion tube connector 162 is the same that of the transfusion tube connector 120 shown in Embodiment 1. As shown in Fig.11, the same as Fig.2, the shape of the transfusion tube connector 162 can fit to the inner female screw of the lock connector 210. Therefore, if the lock connector 210 screws into the transfusion tube connector 162, the lock connector 210 is connected to the transfusion tube connector 162. As a result, iMAP 100 and the transfusion tube 200 are connected each other.

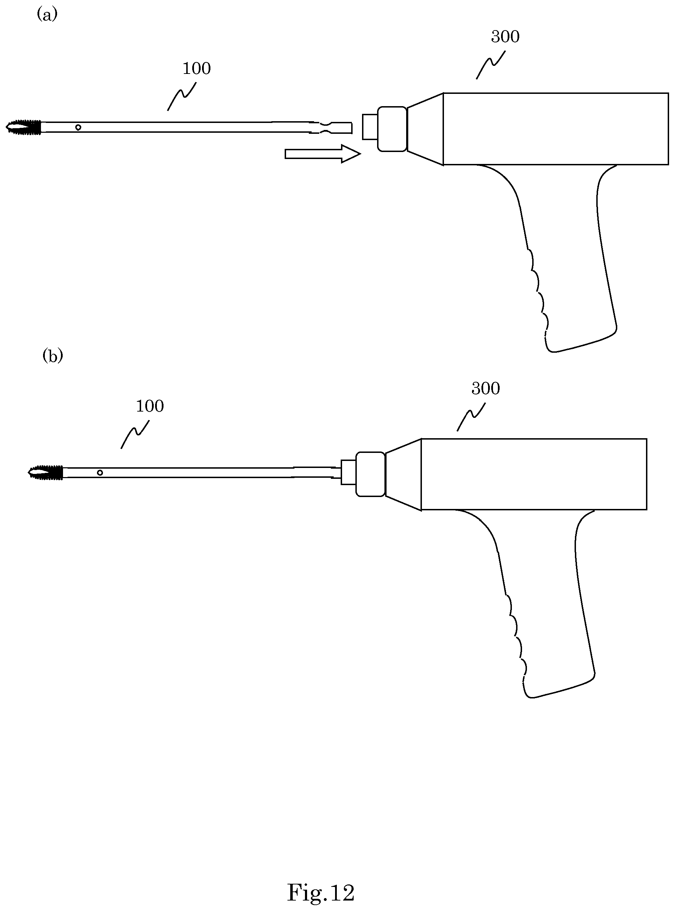

Next, the operation procedure of the iMAP 100a of Embodiment 2 is descried below with referencing to Fig.12 to Fig.14.

As shown in Fig.12 (a), the drill chuck 310 of the electric drill tool 300 is attached to the base end portion of the fixation pin (iMAP pin) 110 of the iMAP 100a. As a result, the iMAP 100a is connected to the electric drill tool 300.

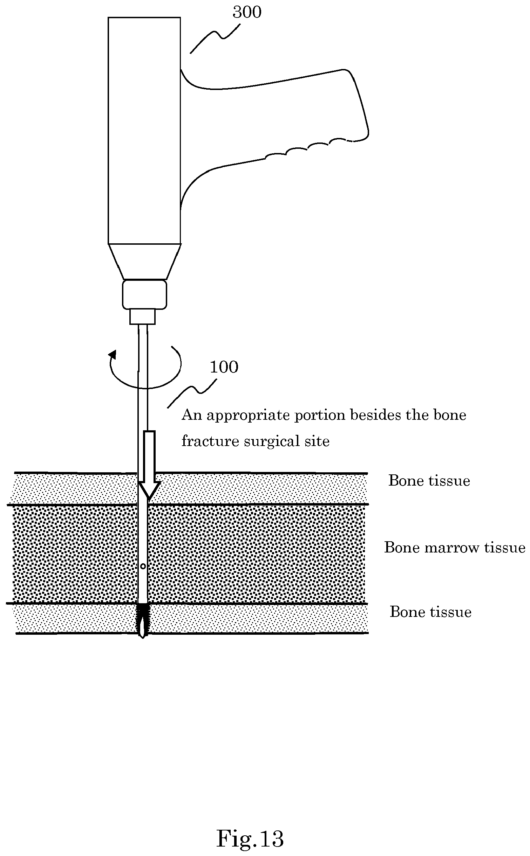

As shown in Fig.13, the iMAP 100a of Embodiment 2 is pierced into the bone besides the patient's bone fracture surgical site. In this example, single iMAP 100a is fixed to the target bone. An operation for a plurality set of iMAP 100 is described in Embodiment 3.

The fixation pin (iMAP pin) 110 has male screw 112 on the head portion for screwing. In advance to pierce the fixation pin (iMAP pin) 110 to the target bone portion, it is preferable that an assist hole which assists the beginning of screwing is opened beforehand by the electric drill tool. This assist hole can assist the fixation pin to be screwed and pierced even if the target bone portion is hard bone tissue. The size of the assist hole should be adjusted to fit the fixation pin.

After fixing the fixation pin (iMAP pin) 110 to the appropriate portion according to the same operation as that of the external skeletal fixator, the transfusion tube attachment 160 is connected to the drill connector 150. The connecting procedure for the transfusion tube attachment 160 to the drill connector 150 is shown Fig.10 (b). The gripping force adjuster 165 of the transfusion tube attachment 160 is pressed to the direction that the taper structure 166 is exit from the gripping lock ball 167 by a finger (the right direction in the drawing). The transfusion tube attachment 160 is inserted from the head portion to the drill connector 150 when the grip force given by the gripping lock ball 167 is small status. After an appropriate insertion, finger is released, then the gripping force adjuster 165 slides to the direction that the taper structure 166 goes into the gripping lock ball 167 by the elastic force of the elastic actuator 164. As a result, the grip force of the gripping lock ball 167 to the dimple 152 becomes large. The drill connector 150 is gripped by the gripping lock ball 167, and the transfusion tube attachment 160 can be attached firmly to the fixation pin (iMAP pin) 110.

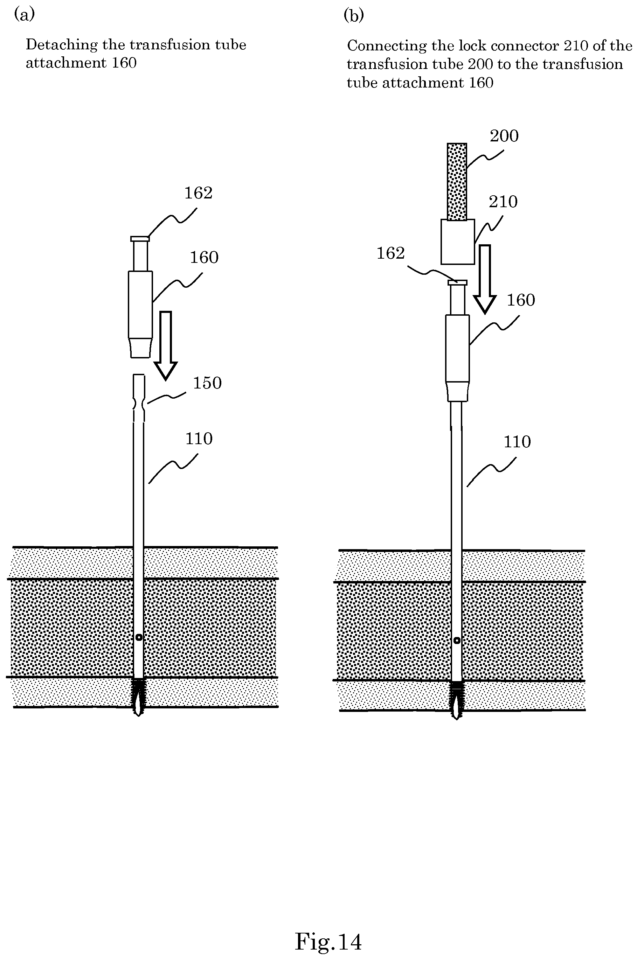

Next, as shown in Fig.14 (b), the lock connector 210 screws into the transfusion tube connector 162, the lock connector 210 is connected to the transfusion tube connector 162. As a result, iMAP 100 and the transfusion tube 200 are connected each other.

Appropriate kind and dose level of antibiotics is perfused to the patient's bone fracture surgical site by the predetermined term and the predetermined speed.

The basic structure and function of the iMAP 100a and the each element of Embodiment 2 are described as shown above. The iMAP 100a of the present invention can be applied to the patient's bone fracture surgical site. Therefore, it is preferable that a required sterilization should be treated on the iMAP as a medical use device.

### Embodiment 3

The device for intra medullary antibiotics perfusion (iMAP) 100 of embodiment 3 according to the present invention is used as both an iMAP and a conventional external fixator to the patient's bone fracture surgical site.

In this example, the material and the shape of the shaft 111 and the screw part 112 of the fixation pin (iMAP pin) 110 of the iMAP 100 is the same as that of the conventional external fixator.

As shown in Embodiment 1 and Embodiment 2, the conventional fixation pin of the external fixator is spread among the orthopedics field. The conventional external fixator is a well-known medical tool which is a standard tool used in an external fixation operation for piercing into a bone besides the patient's bone fracture surgical site and fixing externally. It is said that the conventional external fixator has no problem in the orthopedics field and many valuable insights are accumulated.

The inventor Akihiro MARUO found that the fixation pin (iMAP pin) 110 of the present invention is the same in view of the piercing and fixing to the bone besides the patient's bone fracture surgical site as the conventional external fixator. Therefore, he thought the fixation pin (iMAP pin) 110 of the present invention can be provided as the conventional external fixator.

The inventor Akihiro MARUO took care that the outer diameter of the external fixator is not standardized completely yet, the bridge tool does not always correspond to all external fixators. The inventor Akihiro MARUO has invented that the fixation pin (iMAP pin) 110 of the iMAP 100 of the present invention employs different parts along the axis which diameter size are different.

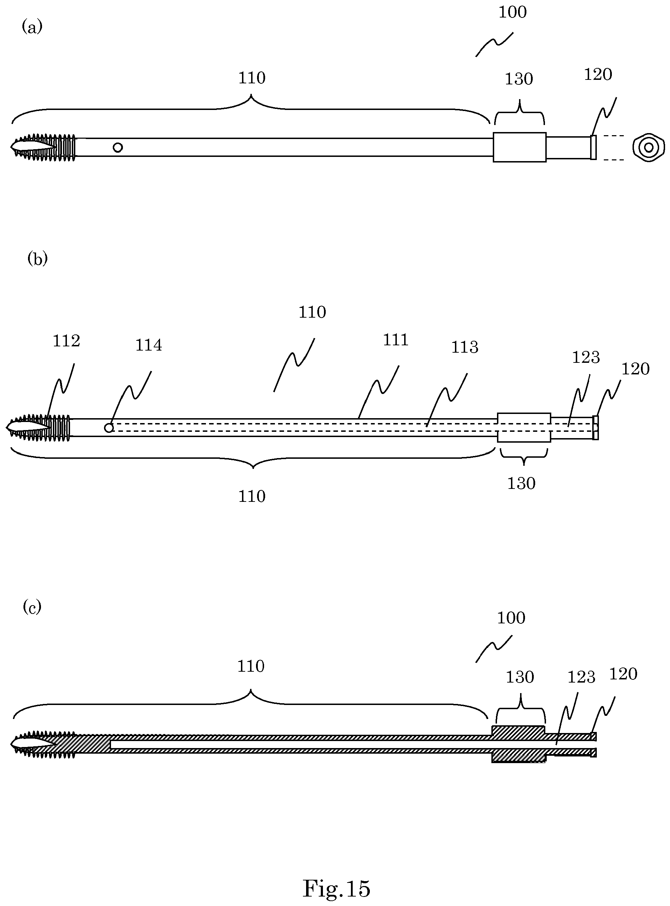

Fig.15 is a schematic structure of the iMAP 100 of Embodiment 1 of the present invention which employs a different part 130 having different diameter. As shown in Fig.15, the outer diameter of the part 130 is larger than that of the other portion of the shaft 111. In short, the diameter R2 of the part 130 is larger than the diameter R1 of the shaft 111. A single iMAP 100 can provides both diameter R1 and diameter R2. Therefore, iMAP 100 can accept various bridge tool, a variety combination of external fixator cluster can be formed.

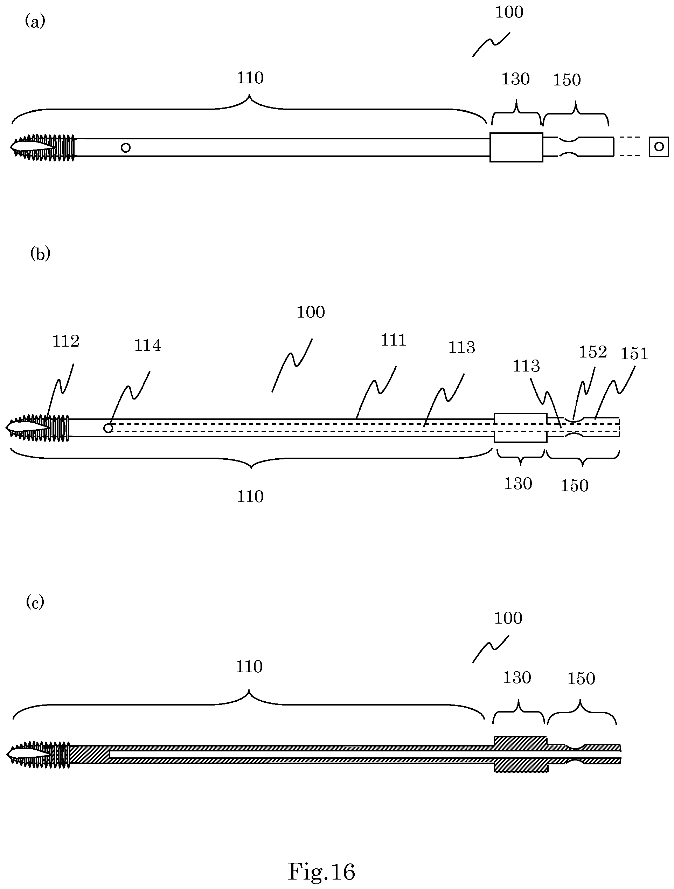

Fig.16 is a schematic structure of the iMAP 100a of Embodiment 2 of the present invention which employs a different part 130 having different diameter. The same as shown in Fig.15, the outer diameter of the part 130 is larger than that of the other portion of the shaft 111. In short, the diameter R2 of the part 130 is larger than the diameter R1 of the shaft 111. A single iMAP 100 can provides both diameter R1 and diameter R2. Therefore, iMAP 100 can accept various bridge tool, a variety combination of external fixator cluster can be formed.

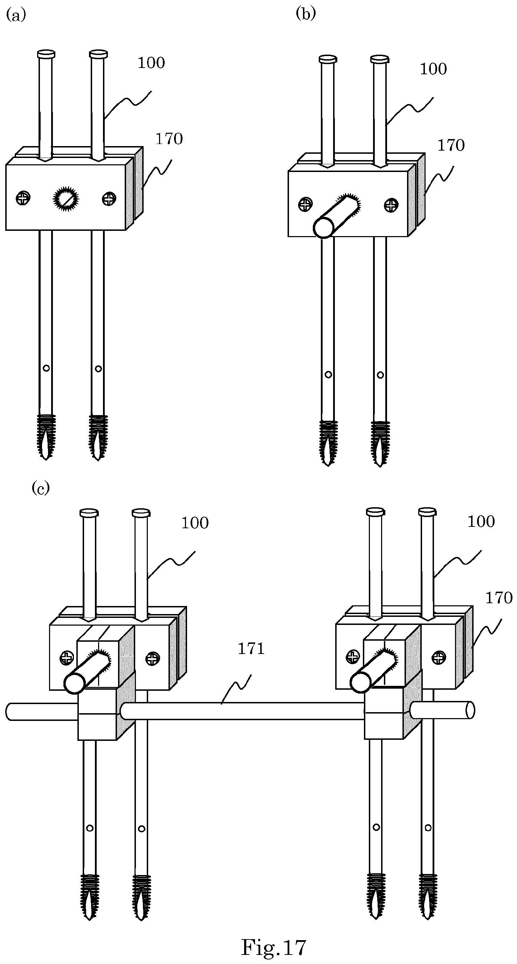

Fig.17 is a schematic view showing the iMAP 100 of embodiment 3 is used as both an iMAP and a conventional external fixator to the patient's bone fracture surgical site. In this example, plurality of iMAP 100 and the bridge tool 170 which bridges between iMAP 100 are employed.

As shown in Fig.17, plural iMAP 100 are pierced and fixed to the bones including the trunk side and the end side of limbs besides the patient's bone fracture surgical site as the external fixator. For example, the case that the bone fracture site is an open tibia fracture is described below. For example, a knee portion is selected for the trunk side and an ankle portion is selected for the end side of limbs as an appropriate portion for the tibia fracture surgical site. Plural iMAP 100 are piercing and fixing to those portions. In advance to pierce the fixation pin (iMAP pin) 110 to the target bone portion, it is preferable that an assist hole which assists the beginning of screwing is opened beforehand by the electric drill tool.

As shown in Fig.17 (a), Two iMAP 100 are piercing to the target portion each other. Then the bridge tool 170 for connecting between the fixation pin (iMAP pin) 110 of these iMAP 100 for keeping the stability. As shown above, plural iMAP 100 are connected via the bridge tool 170 as the cluster of the iMAP 100. The cluster of the iMAP 100 is more stable as a whole than the isolate single iMAP 100. The merits that the iMAP 100 prevents from tilting and being pulled out are obtained.

Fig.17 (b) to Fig.17 (c) shows more complex patterns of the cluster of the iMAP 100. It is possible to bridge between bridge tools 170 via shaft tool 171.

The cluster of the iMAP 100 shown in Fig.17 (c) is a state for providing a scaffold at the scheduled portion according to the instruction of the doctor in charge, for perfusing antibiotics around the bone fracture surgical site.

The state shown in Fig.17 (c) is also a state that external fixator for fixing the bone fracture site externally. Therefore, an operation for the external fixing for the bone fracture site externally and an operation for combining a scaffold at the bone fracture site simultaneously.

A transfuse tube 200 is connected to the base end of an iMAP 100 or plural iMAP 100 selected among a cluster of the iMAP 100. Appropriate kind and dose level is selected, antibiotics can be provided at necessary timing, desirable term and desirable speed via the transfusion tube 200 according to the circumstances of tissue in the bone fracture therapeutic portion.

While some preferable embodiments of the sample storage according to the present invention are described above, it should be understood that various changes are possible, without deviating from the technical scope according to the present invention. Therefore, the technical scope according to the present invention is limited only by the claims attached.

### Industrial applicability

A device for intra medullary antibiotics perfusion according to the present invention can be employed as a device for intra medullary antibiotics perfusion for suppressing the occurrence of the post traumatic deep surgical site infection and various complications.

### Description of the reference numerals

100: Device for intra medullary antibiotics perfusion
110: Fixation pin
111: Shaft
112: Screw part
113: Hollow portion
114: Side face opening
115: Base end opening
120: Transfusion tube connector
130: Different part having different diameter
140: Drill attachment
141: Transfusion tube adapter
142: Chuck connecter
150: Drill attachment
151: Body
152: Dimple portion
160: Transfusion tube attachment
161: Drill connector adapter
162: Transfusion tube connector
163: Detachable mechanism
164: Elastic actuator
165: Gripping force adjuster
166: Taper structure
167: Gripping lock ball
170: Bridge tool

## Claims

1. A device (100) for intra medullary antibiotics perfusion for perfusing an antibiotics to a patient's bone fracture surgical site, applied as an external skeletal fixator comprising;
a fixation pin (110) for externally fixing to a bone by piercing from one side of cortical bone penetrating through bone marrow to the other side of cortical bone;
wherein the fixation pin (110) includes a side face opening (114) at the side of the shaft, which opening is located facing to the bone marrow; a base end opening (115) at the base end part of the fixation pin (110); a hollow structure (113) from the base end opening to the side face opening; and a male screw portion (112) including spiral screw head mounted on the outer circumferential surface of the tip part of the fixation pin (110); and a transfusion tube connector (120) installed to the base end opening of the fixation pin (110), which includes an attachment structure for connecting to a connector (210) attached to the head of a transfusion tube for antibiotics (200),
wherein a perfusion point of antibiotics is limited to the side face opening (114) of the shaft, there is no perfusion point in the male screw portion (112) of the tip part of the fixation pin (110), antibiotics is perfused only into the bone marrow via the side face opening (114) of the shaft from the transfusion tube for antibiotics (200).

2. A device for intra medullary antibiotics perfusion applied as an external skeletal fixator according to claim 1,
wherein the device for intra medullary antibiotics perfusion (100) can connect directly to the transfusion tube for antibiotics (200) via the transfusion tube connector (120).

3. A device for intra medullary antibiotics perfusion (100) applied as an external skeletal fixator according to claim 2, further comprising;
a drill attachment (140) for providing a structure connecting to a drill chuck of an electric drill tool (300) comprising; a transfusion tube adapter (141) for connecting to the transfusion tube connector (120) on the base end part of the fixation pin (110), wherein the adapter shape is the same as that of the lock connector (210) on the head part of the transfusion tube (200); a chuck connecter (142) which can be chucked by the drill chuck of the electric drill tool (300);
wherein the shape of the base end part of the fixation pin (110) can be converted to the shape applicable to the electric drill tool (300) by the drill attachment (140).

4. A device for intra medullary antibiotics perfusion (100) applied as an external skeletal fixator according to claim 1, further comprising;
a drill connector (150) on the base end part, which shape can be applicable to the electric drill tool (300), the fixation pin (110) can be chucked directly by the drill chuck of the electric drill tool (300).

5. A device for intra medullary antibiotics perfusion (100) applied as an external skeletal fixator according to claim 4, further comprising;
a transfusion tube attachment (160) for providing a structure for connecting to the lock connector (210) installed to the head of the transfusion tube for antibiotics (200),
wherein the transfusion tube attachment (160) comprises a drill connector adapter (161) for connecting to the drill connector (150); a transfusion tube connector (120) for connecting to the lock connector (210) on the head part of the transfusion tube (200);
wherein the shape of the base end part of the fixation pin (110) can be converted to the shape applicable to the lock connector (210) on the head part of the transfusion tube (200).

6. A device for intra medullary antibiotics perfusion (100) applied as an external skeletal fixator according to claim 5, wherein the transfusion tube attachment (160) includes a detachable mechanism which can adjust the gripping force between the inner surface of the drill connector adapter (161) and the outer surface of the drill connector (150) of the fixation pin (110),
wherein the detachable mechanism comprises a gripping lock ball (167) installed facing to the outer surface of the drill connector (150) of the fixation pin (110); an elastic actuator; a gripping force adjuster for adjusting the gripping force corresponding to the pressure generated between the gripping lock ball and the outer surface of the drill connector (150) when sliding along a track by the elastic actuator; wherein the structure of the gripping force adjuster contacting to the gripping lock ball has a wedge-shape;
when the gripping force adjuster moves along to the track by the elastic actuator, the gripping force to the drill connector (150) of the fixation pin (110) by the gripping lock ball becomes large by the work of the wedge-shape to the gripping lock ball.

7. A device for intra medullary antibiotics perfusion (100) applied as an external skeletal fixator according to any one of claims 1 to 6, wherein the fixation pin (110) has a wider portion which diameter is larger than that of other portion.

8. A device for intra medullary antibiotics perfusion (100) applied as an external skeletal fixator according to any one of claims 1 to 7, further comprising a bridge connector for connecting between plural fixation pins,
Plural fixation pins are fixed to plural points besides patient's bone fracture surgical site and combined as a cluster of the devices for intra medullary antibiotics perfusion, wherein antibiotics agent is perfused from at least one of the device for intra medullary antibiotics perfusion among the cluster.

9. A device for intra medullary antibiotics perfusion (100) applied as an external skeletal fixator according to any one of claims 1 to 8, wherein the outer shape of the fixation pin (110) is the same as the outer shape of a conventional pin for an external skeletal fixation applied to patient's bone fracture surgical site, the device for intra medullary antibiotics perfusion (100) can apply as the external skeletal fixator providing both external skeletal fixation function and intra medullary antibiotics perfusion function to patient's bone fracture surgical site.

## Patentansprüche

1. Vorrichtung zur intra-medullären Antibiotika-Perfusion (100) zum Perfundieren eines Antibiotikums an eine Operationsstelle einer Knochenfraktur eines Patienten, wobei die Vorrichtung als externer Skelettfixateur verwendet wird, umfassend:
einen Befestigungsstift (110) zur externen Befestigung an einem Knochen mittels Durchstechens von einer Seite eines kortikalen Knochens durch Knochenmark hindurch zur anderen Seite des kortikalen Knochens;
wobei der Befestigungsstift (110) eine dem Knochenmark zugewandte Seitenöffnung (114) an der Seite des Schafts, eine Basisendöffnung (115) am Basisendabschnitt des Befestigungsstifts (110), eine hohle Struktur (113) von der Basisendöffnung zur Seitenöffnung und einen männlichen Schraubenabschnitt (112) mit einem Wendel-Schraubenkopf, der an der äußeren Umfangsfläche des Spitzenabschnitts des Befestigungsstifts (110) angebracht ist, aufweist; und
einen Transfusionsschlauchverbinder (120), der an der Basisendöffnung des Befestigungsstifts (110) angebracht ist, wobei er eine Anbringungsstruktur zum Verbinden mit einem Verbinder (210) aufweist, der an dem Kopf eines Transfusionsschlauchs für Antibiotika (200) angebracht ist,
wobei eine Antibiotika-Perfusionsstelle auf die Seitenöffnung (114) des Schafts begrenzt ist, in dem männlichen Schraubenabschnitt (112) des Spitzenabschnitts des Befestigungsstifts (110) keine Perfusionsstelle ausgebildet ist, Antibiotika in das Knochenmark lediglich über die Seitenöffnung (114) des Schafts von dem Transfusionsschlauch für Antibiotika (200) perfundiert wird.

2. Vorrichtung zur intra-medullären Antibiotika-Perfusion nach Anspruch 1, welche als externer Skelettfixateur verwendet wird,
wobei die Vorrichtung zur intra-medullären Antibiotika-Perfusion (100) über den Transfusionsschlauchverbinder (120) direkt mit dem Transfusionsschlauch für Antibiotika (200) verbunden werden kann.

3. Vorrichtung zur intra-medullären Antibiotika-Perfusion (100) nach Anspruch 2, welche als externer Skelettfixateur verwendet wird, ferner umfassend:
einen Bohreraufsatz (140) zum Bereitstellen einer Struktur zum Verbinden mit einem Bohrfutter eines elektrischen Bohrwerkzeugs (300), umfassend:
einen Transfusionsschlauchadapter (141) zum Verbinden mit dem Transfusionsschlauchverbinder (120) am Basisendabschnitt des Befestigungsstifts (110), wobei die Form des Adapters die gleiche ist wie die des Verschlussverbinders (210) am Kopfabschnitt des Transfusionsschlauchs (200);
einen Bohrfutterverbinder (142), der von dem Bohrfutter des elektrischen Bohrwerkzeugs (300) eingespannt werden kann,
wobei die Form des Basisendabschnitts des Befestigungsstifts (110) durch den Bohreraufsatz (140) in die für das elektrische Bohrwerkzeug (300) geeignete Form umgewandelt werden kann.

4. Vorrichtung zur intra-medullären Antibiotika-Perfusion (100) nach Anspruch 1, welche als externer Skelettfixateur verwendet wird, ferner umfassend:
einen Bohreranschluss (150) am Basisendabschnitt, dessen Form für das elektrische Bohrwerkzeug (300) geeignet ist, wobei der Befestigungsstift (110) direkt von dem Bohrfutter des elektrischen Bohrwerkzeugs (300) eingespannt werden kann.

5. Vorrichtung zur intra-medullären Antibiotika-Perfusion (100) nach Anspruch 4, welche als externer Skelettfixateur verwendet wird, ferner umfassend:
einen Transfusionsschlauchaufsatz (160) zum Bereitstellen einer Struktur zum Verbinden mit dem Verschlussverbinder (210), der am Kopf des Transfusionsschlauchs für Antibiotika (200) angebracht ist,
wobei der Transfusionsschlauchaufsatz (160) einen Bohreranschlussadapter (161) zum Verbinden mit dem Bohreranschluss (150) umfasst; und einen Transfusionsschlauchverbinder (120) zum Verbinden mit dem Verschlussverbinder (210) am Kopfabschnitt des Transfusionsschlauchs (200),
wobei die Form des Basisendabschnitts des Befestigungsstifts (110) in die Form umgewandelt werden kann, die für den Verschlussverbinder (210) am Kopfabschnitt des Transfusionsschlauchs (200) geeignet ist.

6. Vorrichtung zur intra-medullären Antibiotika-Perfusion (100) nach Anspruch 5, welche als externer Skelettfixateur verwendet wird,
wobei der Transfusionsschlauchaufsatz (160) einen abnehmbaren Mechanismus umfasst zum Einstellen der Greifkraft zwischen der Innenfläche des Bohreranschlussadapters (161) und der Außenfläche des Bohreranschlusses (150) des Befestigungsstifts (110),
wobei der abnehmbare Mechanismus umfasst: eine Greifverschlusskugel (167), die der Außenfläche des Bohreranschlusses (150) des Befestigungsstifts (110) zugewandt angeordnet ist; ein elastisches Betätigungselement; eine Greifkrafteinstelleinrichtung zum Einstellen der Greifkraft entsprechend dem Druck, der zwischen der Greifverschlusskugel und der Außenfläche des Bohreranschlusses (150) erzeugt wird, wenn sie durch das elastische Betätigungselement entlang einer Bahn gleitet, wobei die Struktur der Greifkrafteinstelleinrichtung, die mit der Greifverschlusskugel in Kontakt steht, eine Keilform aufweist;
wenn sich die Greifkrafteinstelleinrichtung durch das elastische Betätigungselement entlang der Bahn bewegt, vergrößert sich die Greifkraft der Greifverschlusskugel auf den Bohreranschluss (150) des Befestigungsstifts (110) aufgrund der Wirkung der Keilform auf die Greifverschlusskugel.

7. Vorrichtung zur intra-medullären Antibiotika-Perfusion (100) nach einem der Ansprüche 1 bis 6, welche als externer Skelettfixateur verwendet wird,
wobei der Befestigungsstift (110) einen breiteren Abschnitt aufweist, dessen Durchmesser größer ist als der des anderen Abschnitts.

8. Vorrichtung zur intra-medullären Antibiotika-Perfusion (100) nach einem der Ansprüche 1 bis 7, welche als externer Skelettfixateur verwendet wird,
ferner einen Brückenverbinder aufweisend zum Verbinden mehrerer Befestigungsstifte miteinander,
wobei die mehreren Befestigungsstifte an mehreren Stellen neben einer Operationsstelle einer Knochenfraktur eines Patienten befestigt werden und als eine Gruppe von Vorrichtungen zur intra-medullären Antibiotika-Perfusion verbunden sind, wobei von mindestens einer der Vorrichtungen zur intra-medullären Antibiotika-Perfusion der Gruppe ein Antibiotika-Wirkstoff perfundiert wird.

9. Vorrichtung zur intra-medullären Antibiotika-Perfusion (100) nach einem der Ansprüche 1 bis 8, welche als externer Skelettfixateur verwendet wird,
wobei die äußere Form des Befestigungsstifts (110) die gleiche ist wie die äußere Form eines herkömmlichen Stifts für eine externe Skelettfixierung, die an einer Operationsstelle einer Knochenfraktur eines Patienten angebracht wird, und die Vorrichtung zur intra-medullären Antibiotika-Perfusion (100) als der externe Skelettfixateur angewendet werden kann, der sowohl die Funktion der externen Skelettfixierung als auch die Funktion der intra-medullären Antibiotika-Perfusion an der Operationsstelle der Knochenfraktur des Patienten bereitstellt.

## Revendications

1. Dispositif (100) de perfusion intramédullaire d'antibiotique destiné à perfuser un antibiotique dans le site chirurgical de fracture osseuse d'un patient, appliqué en tant que dispositif de fixation squelettique externe, comprenant :
une broche de fixation (110) de fixation externe à un os par perçage d'un côté d'os cortical, en pénétrant la moelle osseuse, à l'autre côté d'os cortical ;
dans lequel la broche de fixation (110) comprend une ouverture de face latérale (114) au niveau du côté de la tige, laquelle ouverture est située en regard de la moelle osseuse ; une ouverture d'extrémité de base (115) au niveau de la partie d'extrémité de base de la broche de fixation (110) ; une structure creuse (113) allant de l'ouverture d'extrémité de base à l'ouverture de face latérale ; et une partie de vis mâle (112) comprenant une tête de vis en spirale montée sur la surface circonférentielle extérieure de la partie de pointe de la broche de fixation (110) ; et
un raccord (100) de tube de transfusion installé sur l'ouverture d'extrémité de base de la broche de fixation (110), qui comprend une structure de fixation à des fins de raccordement à un raccord (210) fixé à la tête d'un tube de transfusion d'antibiotique (200),
dans lequel un point de perfusion d'antibiotique est limité à l'ouverture de face latérale (114) de la tige, aucun point de perfusion n'étant présent dans la partie de vis mâle (112) de la partie de pointe de la broche de fixation (110), l'antibiotique n'étant perfusé dans la moelle osseuse que par le biais de l'ouverture de face latérale (114) de la tige à partir du tube de transfusion d'antibiotique (200).

2. Dispositif de perfusion intramédullaire d'antibiotique appliqué en tant que dispositif de fixation squelettique externe selon la revendication 1,
dans lequel le dispositif de perfusion intramédullaire d'antibiotique (100) peut être raccordé directement au tube de transfusion d'antibiotique (200) par le biais du raccord (120) de tube de transfusion.

3. Dispositif de perfusion intramédullaire d'antibiotique (100) appliqué en tant que dispositif de fixation squelettique externe selon la revendication 2, comprenant en outre :
un accessoire de foret (140) destiné à fournir une structure de prise avec un mandrin porte-foret d'une perceuse électrique (300), comprenant : un adaptateur (141) de tube de transfusion de raccordement au raccord (120) de tube de transfusion sur la partie d'extrémité de base de la broche de fixation (110), dans lequel la forme d'adaptateur est la même que celle du raccord de verrouillage (210) situé sur la partie de tête du tube de transfusion (200) ; un raccord (142) de mandrin qui peut être pris en mandrin par le mandrin porte-foret de la perceuse électrique (300) ;
dans lequel la forme de la partie d'extrémité de base de la broche de fixation (110) peut être convertie en la forme pouvant être appliquée à la perceuse électrique (300) par l'accessoire de foret (140).

4. Dispositif de perfusion intramédullaire d'antibiotique (100) appliqué en tant que dispositif de fixation squelettique externe selon la revendication 1, comprenant en outre :
un raccord de foret (150) situé sur la partie d'extrémité de base, dont la forme peut être appliquée à la perceuse électrique (300), la broche de fixation (110) pouvant être prise en mandrin directement par le mandrin porte-foret de la perceuse électrique (300).

5. Dispositif de perfusion intramédullaire d'antibiotique (100) appliqué en tant que dispositif de fixation squelettique externe selon la revendication 4, comprenant en outre :
un accessoire (160) de tube de transfusion destiné à fournir une structure de raccordement au raccord de verrouillage (210) installé dans la tête du tube de transfusion d'antibiotique (200),
dans lequel l'accessoire (160) de tube de transfusion comprend un adaptateur (161) de raccord de foret permettant un raccordement au raccord de foret (150) ; un raccord (120) de tube de transfusion permettant un raccordement au raccord de verrouillage (210) situé sur la partie de tête du tube de transfusion (200) ;
dans lequel la forme de la partie d'extrémité de base de la broche de fixation (110) peut être convertie en la forme pouvant être appliquée au raccord de verrouillage (210) situé sur la partie de tête du tube de transfusion (200).

6. Dispositif de perfusion intramédullaire d'antibiotique (100) appliqué en tant que dispositif de fixation squelettique externe selon la revendication 5, dans lequel l'accessoire (160) de tube de transfusion comprend un mécanisme démontable qui permet de régler la force de saisie entre la surface intérieure de l'adaptateur (161) de raccord de foret et la surface extérieure du raccord de foret (150) de la broche de fixation (110),
dans lequel le mécanisme démontable comprend une bille de verrouillage de saisie (167) installée en regard de la surface extérieure du raccord de foret (150) de la broche de fixation (110) ; un actionneur élastique ; un dispositif de réglage de force de saisie permettant de régler la force de saisie correspondant à la pression générée entre la bille de verrouillage de saisie et la surface extérieure du raccord de foret (150) lors du coulissement le long d'un chemin de guidage par l'actionneur élastique ;
dans lequel la structure du dispositif de réglage de force de saisie contactant la vis de verrouillage de saisie a une forme de coin ;
lorsque le dispositif de réglage de force de saisie est déplacé le long du chemin de guidage par l'actionneur élastique, la force de saisie appliquée au raccord de foret (150) de la broche de fixation (110) par la bille de verrouillage de saisie augmente du fait du travail de la forme de coin sur la bille de verrouillage de saisie.

7. Dispositif de perfusion intramédullaire d'antibiotique (100) appliqué en tant que dispositif de fixation squelettique externe selon l'une quelconque des revendications 1 à 6, dans lequel la broche de fixation (110) comporte une partie plus large dont le diamètre est plus grand que celui d'une autre partie.

8. Dispositif de perfusion intramédullaire d'antibiotique (100) appliqué en tant que dispositif de fixation squelettique externe selon l'une quelconque des revendications 1 à 7, comprenant en outre un raccord en pont permettant un raccordement entre plusieurs broches de fixation,
plusieurs broches de fixation sont fixées en plus à plusieurs points du site chirurgical de fracture osseuse du patient et sont combinées sous la forme d'un groupement des dispositifs de perfusion intramédullaire d'antibiotique, dans lequel un agent antibiotique est perfusé à partir d'au moins un dispositif des dispositifs de perfusion intramédullaire d'antibiotique du groupement.

9. Dispositif de perfusion intramédullaire d'antibiotique (100) appliqué en tant que dispositif de fixation squelettique externe selon l'une quelconque des revendications 1 à 8, dans lequel la forme extérieure de la broche de fixation (110) est la même que la forme extérieure d'une broche classique de fixation squelettique externe appliquée au site chirurgical de fracture osseuse du patient, le dispositif de perfusion intramédullaire d'antibiotique (100) peut être appliqué en tant que le dispositif de fixation squelettique externe assurant à la fois une fonction de fixation squelettique externe et une fonction de perfusion intramédullaire d'antibiotique au site chirurgical de fracture osseuse du patient.
